# EUROPEAN PATENT SPECIFICATION

(11) **EP 2 146 660 B1**
(45) Date of publication and mention of the grant of the patent: **29.07.2015**
(21) Application number: 07736794.4
(22) Date of filing: 19.04.2007
(51) Int. Cl.: A61C 1/07, A61C 17/20, A61B 17/32, B06B 3/00

(54) **ULTRASOUND FREQUENCY RESONANT DIPOLE FOR MEDICAL USE**
ULTRASCHALLFREQUENZ-RESONANZ-DIPOL FÜR MEDIZINISCHE ANWENDUNGEN
DIPÔLE RÉSONNANT À FRÉQUENCE D'ULTRASONS POUR UNE UTILISATION MÉDICALE

(43) Date of publication of application: 27.01.2010
(73) Proprietor: Mectron S.p.A., 16042 Carasco GE (IT)
(72) Inventor: VERCELLOTTI, Domenico, I-16039 Sestri Levante (Genova) (IT); BIANCHETTI, Fernando, I-16043 Chiavari (IT); CARDONI, Andrea, G3 8LX Glasgow (GB)
(74) Representative: Petruzziello, Aldo
(86) International application number: PCT/IT2007/000290
(87) International publication number: WO 2008/129570

(56) References cited:
- EP-A- 0 594 541
- EP-A- 0 914 809
- WO-A-2005/009256
- WO-A-2007/101362
- DE-U1- 20 113 692
- US-A- 3 015 961

## Description

The present invention refers to a resonant dipole or ultrasound sonotrode for medical applications and in particular for surgical procedures and for dental surgery.

Ultrasound handsets or handpieces used to cut bone tissue, perforate the bone (insertion of implants), and remove plaque are known to the art in the surgical and orthodontic fields. Such an ultrasound handset has been described in patents EP 0 914 809 and US 6.695.847 in the name of the same applicant.

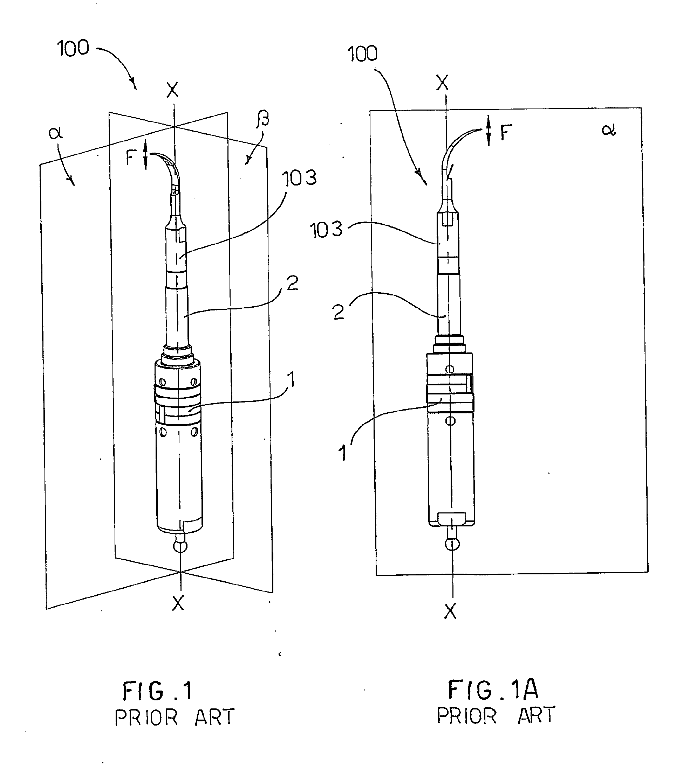

Figures 1 and 1a show an ultrasound handset according to the prior art, designated as a whole with reference numeral 100. The handset 100 comprises:
1) a mechanical vibration generating element 1, consisting of an ultrasound transducer, which can be of the piezoelectric type, that is, which transforms the alternating electrical field applied to piezoelectric ceramics into a mechanical vibration at ultrasound frequency in the range of 22 to 30 kHz; and
2) a vibration amplifying element 2, commonly known as a booster or horn, which can have a straight, tapered, exponential or stepped profile, which is connected coaxially to the transducer 1 to amplify the vibration amplitude; and
3) a sonotrode 103 connected coaxially to the vibration amplifier 2, which can itself (according to the shape) serve as the working "tool", for example an insert for perforating bone (preparation of the implant site), for cutting bone, and for removal of plaque.

The cylindrical ultrasound transducer 1 (of the piezoelectric type or also of the magnetostriction type) provides a substantially two-way movement along the longitudinal axis X-X of the transducer/amplifier assembly, as shown in Figures 1 and 1A. Consequently, in the above described configuration, the sonotrode/tool 103 is subjected to a prevalently two-way movement along the longitudinal axis X-X of the transducer 1 with minimal transverse vibration.

Essentially, the oscillation to which the end of the sonotrode is subjected has a prevalent axial component (to-and-from movement along the arrows F, which gives the sonotrode 103 its working function) and a minimal, almost non-existent perpendicular component with respect to the longitudinal axis X-X of the transducer.

In some types of surgical procedures, such as for example the creation of an implant site, it is desirable for the axis of vibration of the tool to be rotated by a well-defined angle (preferably about 105°) with respect to the axis of vibration of the transducer, which is disposed in the handle of the handset.

Patent US 5.426.341 teaches that to rotate the axis of vibration of the tool with respect to the axis of vibration of the transducer, use can be made of a sonotrode consisting of a continuous annular element. Such a sonotrode makes it possible to obtain a two-way ultrasonic vibration of the tool, rotated 90° with respect to the axis of the piezoelectric transducer, by exploiting a four-node flexural mode of the annular element of the sonotrode. Moreover, a rotation of the two-way axis of vibration by an angle of 120° can be obtained by exciting a different flexural harmonic of the annular element and exploiting a six-node flexural mode of the annular element.

However, such a type of sonotrode is not suitable for surgical procedures, such as, for example, precision procedures in difficult-to-access sites, such as the oral cavity.

If an annular element of such a size as to allow insertion into the oral cavity is used - that is, with a an outside diameter of less than 20 mm - said annular element begins to vibrate with a flexural vibration mode at a frequency about 3 kHz greater than the frequency of the longitudinal vibration mode of the transducer, which is about 27 kHz. In this case the hammering action of the tool is not very efficient and is not suitable for a surgical procedure. In fact, it must be considered that in order for coupling between the two vibration modes (longitudinal vibration of the transducer and flexural vibration of the sonotrode) to take place with the maximum efficiency, the resonance frequencies of each excited mode must coincide.

Consequently, to make the frequency of the flexural mode of the sonotrode converge (decrease) with the frequency (27 kHz) of the longitudinal mode of the transducer, suitable geometric dimensioning of the sonotrode is necessary. In practice, the inside and/or outside diameter of the sonotrode must be increased to a pre-set optimal value well over 20 mm. These size constraints are in conflict with the requirements for use of the sonotrode in surgery, where the outside diameter and the thickness of the annular element of the sonotrode are dictated by the need to combine the requirement of having a device of limited size with that of operating within the fatigue limit of the material.

Moreover, if the tool is disposed on the annular element with an axis inclined 105° with respect to the axis of the transducer, the percussion efficiency of the tool is extremely limited. In fact, the sonotrode described in US 5.426.341 ensures the maximum efficiency when the tool is disposed with an axis inclined by 90° (four-node flexural mode) or 120° (six-node flexural mode) with respect to the axis of the transducer. These tool positions are not suitable for surgical procedures.

WO2005/009256 A2 **discloses a treatment instrument embodied as vibrating body and coupled to a vibrating drive or comprises a coupling surface on which a sonotrode may be placed.**

Object of the present invention is to overcome the drawbacks of the prior art, by providing an ultrasound sonotrode that is adapted to allow rotation of the ultrasound vibration axis of the tool applied thereto by a well-defined angle with respect to the ultrasound vibration axis of the ultrasound transducer of the handset.

Another object of the present invention is to provide such a sonotrode that ensures high efficiency of the vibrations of the tool and at the same time is of limited size, such as to allow use thereof in surgery at difficult-to-access sites

These objects are achieved in accordance with the invention with the characteristics set forth in appended independent claim 1.

Advantageous embodiments of the invention are apparent from the dependent claims.

The sonotrode according to the invention comprises a tang designed to be connected to a handset comprising an ultrasound transducer that vibrates at ultrasound frequency with a longitudinal axis of vibration coaxial with the axis of the tang, an annular element connected to said tang, and a tool disposed on said annular element and having an axis of vibration inclined with respect to the axis of vibration of the transducer. The annular element is discontinuous and has an aperture **disposed along the continuation of the axis of the transducer, in a position diametrically opposite the attachment of the tang to the annular element of the sonotrode.**

Provision of this aperture ensures that, in the case of a small-sized sonotrode, adapted to be inserted in an oral cavity, the oscillation frequency of the two-way movement of the tool connected to the sonotrode corresponds exactly to be vibration frequency generated in the piezoelectric ultrasound transducer to which the sonotrode is connected, thus ensuring the maximum efficiency of the hammering effect of the tool.

In particular, said aperture is disposed in a suitable position on the annular element so as to generate four-node flexural vibrations of the annular element, wherein the greatest vibration amplitude is obtained in the intermediate position between two nodes which corresponds to an angle of about 105° with respect to the axis of vibration of the transducer. Consequently, the tool can be disposed on the annular element so that its axis of vibration is inclined, with respect to the axis of vibration of the transducer, by an angle between 80° and 140°, preferably 105°. In this manner the maximum efficiency of the hammering effect of the tool is ensured precisely when the tool is disposed in an optimal position to perform surgical procedures.

For the above-mentioned reasons, the sonotrode according to the invention is particularly suitable to be used in the medical field, in the field of bone and tooth implants. In this field a handset comprising said sonotrode can be used to insert into the prepared bone tissue (implant site) new types of implants (screws/posts, etc) like those, for example described in US 7.008.226, incorporated herein as a reference.

Provision of particular cutting tools (per se known and therefore not described) in the sonotrode according to the invention, allows (thanks to the resulting percussion movement) preparatory holes to be made in the bone tissue (implant site), with a final action of smoothing and finishing, for the insertion of screws, posts, etc used in bone implantology.

Further characteristics of the invention will be made clearer by the detailed description that follows, referring to purely exemplifying and therefore non limiting embodiments thereof, illustrated in the appended drawings, wherein:
Figures 1 and 1A show respectively a perspective view and a side view of an ultrasound handset according to the prior art;
Figures 2 and 2A show respectively a perspective view and a side view of an ultrasound handset provided with a sonotrode according to the invention;
Figures 3 and 3A show respectively a perspective view and a side view of a sonotrode **not** according to the invention in which the hammer tool has been omitted;
Figures 4 and 4A show respectively a perspective view and a side view of a variant **not according to the invention** of the sonotrode of figures 3 and 3A;
Figure 5 is a perspective view of the sonotrode of figure 4 in which a hammer shaped tool **according to the invention** has been added;
Figures 6A, 6B and 6C are perspective views of three different embodiments of the hammer;
Figures 7 and 7A show respectively a perspective view and a side view of the sonotrode of Figures 3 and 3A to which the hammer of figure 6B has been added;
Figures 8 e 8A show respectively a perspective view and a side view of the sonotrode of

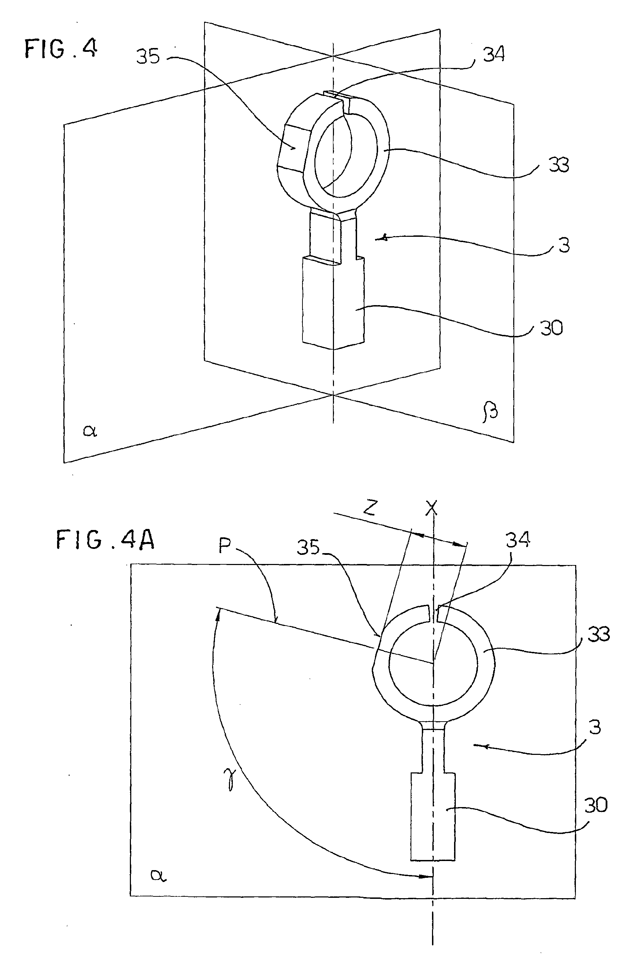

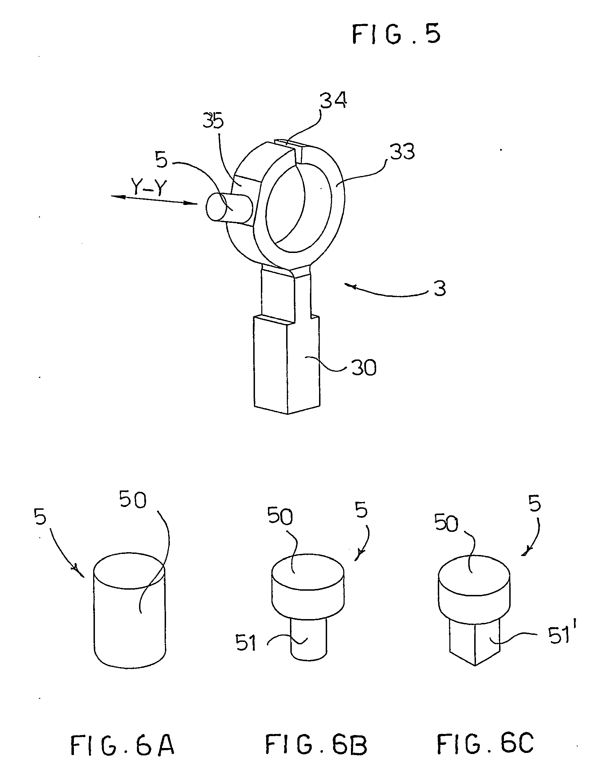

Figures 4 and 4A to which the hammer of Figure 6B has been added;

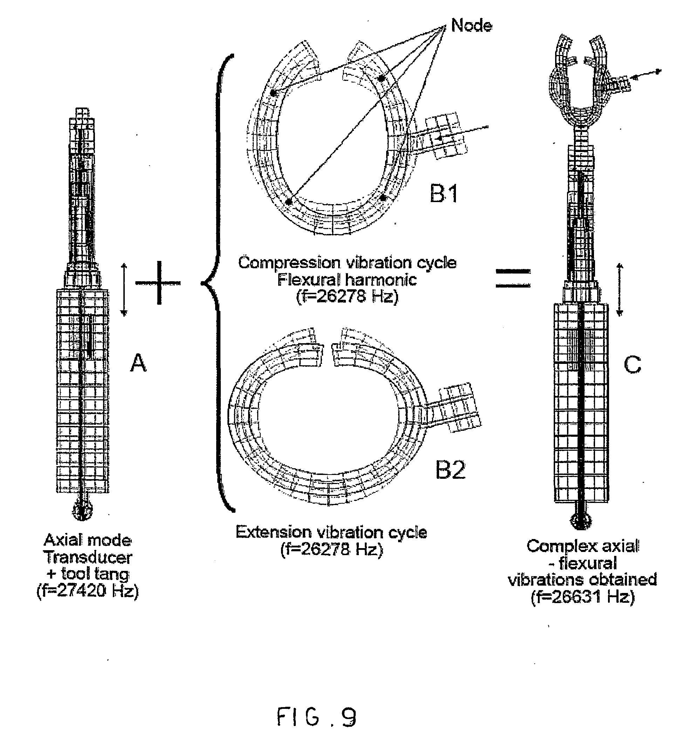

Figures 9 and 9A show respectively a perspective view and a side view of the sonotrode;

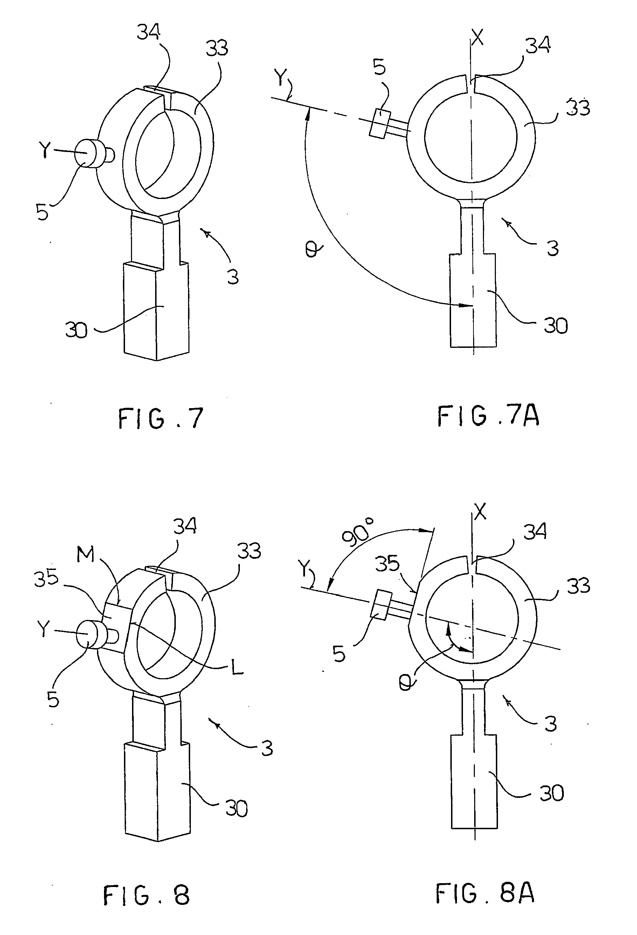

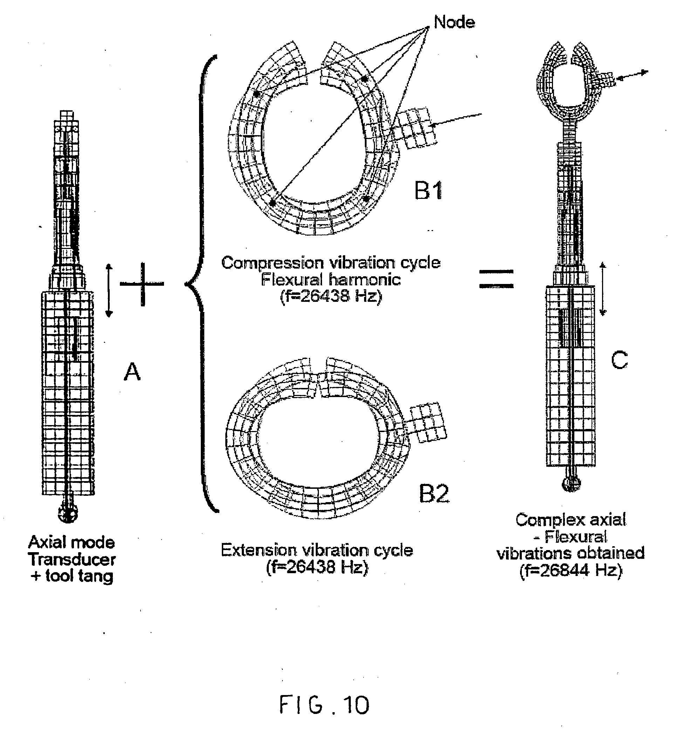

and Figure 10 is a scheme of the finite element analysis performed on an ultrasound handset provided with the sonotrode of Figures 8 and 8A.

The sonotrode according to the invention will be described with the aid of the figures.

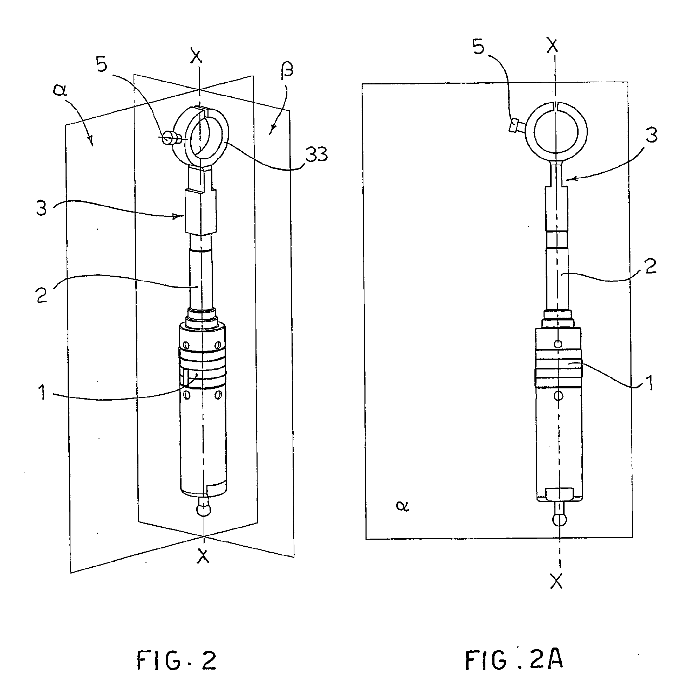

Figures 2 and 2A show an ultrasound handset comprising an ultrasound transducer 1 that vibrates at ultrasound frequency (24 - 29 kHz, preferably 27.0 kHz) along its axis of symmetry X-X and a booster 2 connected coaxially to the transducer 1 to amplify the amplitude of vibration. A sonotrode according to the invention, indicated as a whole with reference numeral 3, is connected to the booster 2. The sonotrode 3 comprises a tool 5 which is preferably made integrally with the sonotrode 3. However, the tool 5 can be applied, as a separate element, to a special attachment of the sonotrode, so as to be replaceable. The tool 5 preferably consists of a percussion element, known as a hammer, adapted to perform a hammering percussion on the surgical site.

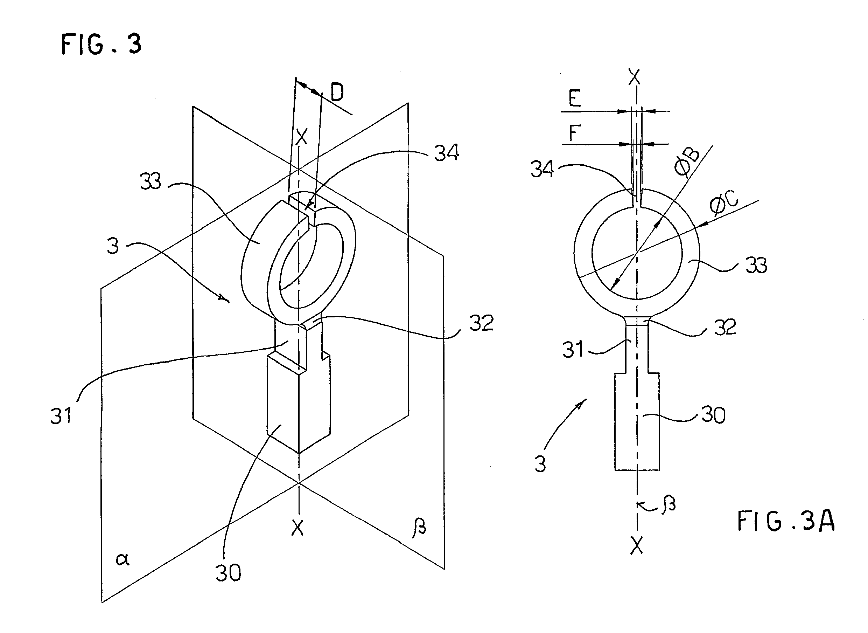

With reference to Figures 3 and 3A **showing an embodiment in which the tool 5 has been omitted and thus not according to the invention as claimed,** the sonotrode 3 comprises a tang composed of two parts 30, 31 coaxial with each other and designed to be disposed coaxially to the axis X-X of the transducer 1 and booster 2 assembly (Figures 2, 2A). The first part 30 of the tang has a square cross-section (but in another configuration it can be circular in section, for example) and is designed to be connected to the booster 2 of the piezoelectric transducer (Figures 2, 2A). For this purpose, the first part 30 of the tang has an internal thread adapted to be screwed onto the external thread of the booster 2, so as to fix the whole sonotrode 3 to the booster of the transducer.

The second part 31 of the tang has a rectangular cross-section (but in another configuration it can be circular in section, for example) with a smaller surface than the section of the first part 30. The second part 31 of the tang is connected directly to the first part 30. The second part 31 of the tang is connected at its distal end, by means of a tapered transition element 32, to an open annular element 33 with a solid cross-section, preferably rectangular.

The annular element 33 has:
- an inside diameter (B) between 4.0 mm and 16 mm, preferably between 8.00 mm and 12.00 mm
- an outside diameter (C) between 6 mm and 20 mm, preferably between 10 mm and 16 mm, and
- a thickness (D) between 2 mm and 8 mm, preferably between 3 mm and 6 mm.

The annular element 33 lies on a plane α and extends symmetrically with respect to the plane β at right angles to the plane α and containing the axis (X-X) of the transducer. The annular element 33 has in its upper part, corresponding to the continuation of the axis of symmetry X-X of the transducer, an aperture 34 (gap, break). The aperture 34 is disposed in a diametrically opposite position with respect to the attachment to 32 of the tang of the annular element.

The width of the aperture 34 increases outwardly. Therefore, on the inside diameter of the annular element, the aperture 34 has a width (F) between 0.3 mm and 2.5 mm, preferably between 0.6 mm and 1.8 mm, whereas on the outside diameter of the annular element, the aperture 34 has a width (E) between 0.5 mm and 3 mm, preferably between 0.8 mm and 2 mm.

In the embodiment of the sonotrode 3 illustrated in figures 3, 3A, **which is not covered by the claims,** the outside diameter of the annular element 33 is kept constant.

In figures 4 and 4A a variant of the sonotrode 3 **also not covered by the claims** is illustrated, wherein a flat surface 35, preferably rectangular in section, is formed by milling on the outer surface of the annular element 33. The straight line P perpendicular to the flat surface 35 forms an angle γ between 80° and 140°, preferably 105°, with respect to the axis of symmetry X-X of the transducer. The flat surface 35 is at a distance (Z) from the centre of the annular element comprised between 3 mm and 10 mm, preferably between 5 mm and 8 mm.

Figure 5 shows the hammer 5 disposed on the flat surface 35 of the annular element. The axis Y-Y of the hammer 5 is at right angles to the flat surface 35.

The hammer 5 can have different geometrical structures, as shown in figures 6A, 6B and 6C. The different geometries are related to the vibration amplitude that is to be obtained on the hammer of 5 and to the consequent material fatigue stress limit in the area of attachment of the hammer 5 to the ring 33. Different geometrical structures of the hammer 5, with a circular or square section, are illustrated in the figures; however, the hammer 5 may also have other types of sections. Moreover, even if a hammer 5 formed integrally in a single piece with the annular element 33 is illustrated in the figures, it is obvious that in place of the hammer, an attachment adapted to receive any per se known tool 5 for applications with ultrasound handsets can be provided on the annular element 33.

Figure 6A shows a hammer 5 with a cylindrical structure 50.

Figure 6B shows a hammer 5 having a structure composed of two cylindrical coaxial parts 50, 51 having different sections. The larger diameter part 50 is the percussion part designed to hammer on the surgical site. The smaller diameter part 51 is the part for attachment to the annular element 33.

Figure 6C shows a hammer 5 formed by a cylindrical percussion part 50 and a parallelepiped attachment part 51' with a square section with sides smaller than the diameter of the cylindrical percussion part 50.

The configurations of figures 6B and 6C allow a high vibration amplitude to be obtained and the mechanical stress at the base of attachment between the hammer and the ring to be contained within the fatigue limits of the sonotrode material.

The diameter of the cylindrical percussion part 50 is between 1.0 mm and 5.0 mm, preferably 3 mm. The attachment part 51, 51', on the other hand, has a diameter or side measuring between 0.5 mm and 2.5 mm, preferably 1.5 mm. The hammer 5 in any structure has an overall length ranging between 1.5 mm and 7.0 mm, preferably 3 mm.

With reference to Figures 7 and 7A, if the annular element 33 has a constant outside diameter, the hammer 5 is positioned on the outer surface of the annular element 33 so that its longitudinal axis of symmetry Y-Y forms an angle ϑ between 80° - 140°, preferably 95° and 115°, more preferably 105°.

With reference to Figures 8 and 8A, if the annular element 33 has a flat portion 35; the hammer 5 is positioned on the flat portion 35, at right angles thereto. In this configuration the hammer 5 is positioned asymmetrically with respect to the longer side L of the flat surface 35 and symmetrically with respect to the shorter side M of the flat surface 35 (however it is possible to have a structure with the hammer 5 positioned symmetrically with respect to both the above mentioned sides of the flat surface 35). Obviously, in this case also, the hammer 5 has an axis Y perpendicular to the flat surface 35 and therefore inclined by angle ϑ between 80° and 140°, preferably 95°-115°, more preferably 105°, with respect to the axis of symmetry X-X of the transducer.

In this manner, the hammer 5, whatever its geometrical structure (previously described), and in whatever type of sonotrode structure (previously described) it is applied, transmits to the head of the implant on which it rests, a two-way/alternating, hammering ultrasonic vibration, acting along its own longitudinal axis of symmetry Y-Y. The hammering effect allows insertion into the implant site of new types of implants (screws/posts, etc) like those described, for example, in US 7.008.226.

Thanks to the provision of the gap 34 in the annular element 33, the sonotrode 3 according to the invention is able to transfer (transform) the two-way oscillation along an axis (X-X) into a two-way oscillation of the same frequency and significant amplitude, acting along a range of axes (Y-Y) rotated by an angle ϑ of about 105° with respect to the reference axis (X-X).

To understand the principle by which rotation of the two-way axis of rotation takes place, using the above described geometrical configuration, it is necessary to divide the entire vibrating system into two subsystems. The first subsystem consists of the piezoelectric transducer 1, the booster 2, and the tang 30, 31, 32 of the sonotrode, and the second subsystem is formed by the annular element 33 with the aperture 34.

Initially an alternating electric current is applied to the piezoelectric ceramics of the transducer 1. In the piezoelectric ceramics the alternating electric current is converted into a mechanical oscillation, thanks to a reverse piezoelectric effect. Thus, in the transducer-booster-tang subsystem a longitudinal stationary wave is produced. The tang 30, 31, 32 of the sonotrode is dimensioned so that an excited longitudinal mode occurs along the axis X-X, preferably at a frequency around 27 kHz.

Figures 9 and 10 show a finite element model illustrating the longitudinal (axial) mode of the first subsystem (transducer-booster-tang) denoted by A.

The second subsystem consisting of the annular element 33 with the apertura 34 and the hammer 5 is in turn dimensioned so that it has a flexural vibration mode at a frequency preferably around 27 kHz and with a geometry that allows application thereof in a surgical site, for example insertion inside the oral cavity. The harmonic of the flexural mode of interest for this application is that with four nodes and it is represented by the finite element models denoted by B1 and B2 in Figures 9 and 10, respectively, for the annular element with a constant outside diameter and with a flat surface.

The longitudinal (axial) mode has its point of greatest amplitude (antinode) at the distal end of the tang connected to the annular element. The same point forms an antinode in the flexural vibration mode of the annular element with aperture. Thus a flexural oscillation of the annular element of the sonotrode is activated by the longitudinal vibration of the transducer-booster-tang subsystem.

The resulting vibration (that is the vibration rotated with respect to the axis X-X of the transducer) of a sufficient amplitude to allow insertion of the implant into the bone can thus be obtained through the combination of two families of vibration modes: that is, the longitudinal mode (along the longitudinal axis of symmetry X-X of the transducer) and the flexural mode of the annular element with aperture and incorporated protuberance or hammer. The complex vibration mode (longitudinal and flexural) of the entire system is represented by the finite element models denoted by C and illustrated in Figures 9 and 10, respectively, for the annular element with a constant outside diameter and with a flat surface.

In order for the two families of modes to be combined correctly, giving rise to the complex vibration necessary for the operation, the vibrating system must be dimensioned so that the types of (simultaneously) excited modes are tuned to the same resonant frequency. The flexural vibration excited in the annular element with aperture and incorporated protuberance or hammer is in turn translated into a two-way vibration, also around 27 kHz, along the axis Y-Y of the hammer.

Therefore, the compression and extension vibration cycles acting on the hammer produce a hammering effect. This hammering effect applied to the head of an implant allows the insertion of said implant into the bone tissue (implantation site) and simultaneous melting of the bioabsorbable plastic. For the above described uses the sonotrode 3 must therefore be of limited size, so as to have easy access to the surgical site, for example the oral cavity.

As is obvious from the finite element analyses, the configuration with a constant outside diameter of the ring 33 is used when it is necessary to give the hammer 5 high-amplitude vibrations, whilst maintaining the mechanical stress values at the base of the hammer-ring attachment point within the fatigue limits of the material. The configuration of the ring 33 with a flat surface 35 formed on the outer surface of the ring, on the other hand, allows a perfectly linear and absolutely axial vibration of the hammer 5 to be obtained with a consequent uniformity of vibration at the points of contact with the implant. The vibration amplitudes given to the hammer in this configuration are smaller than those that can be reached by means of the configuration without a flat surface.

The final object of the invention is that of obtaining an alternating movement of "significant" amplitude acting along the axis Y-Y of the hammer 5, inclined with respect to the axis X-X of the transducer. The desired movement of the hammer 5 and its maximum efficiency, in terms of amplitude, has been obtained by making the longitudinal oscillation frequency of the transducer-booster-tang subsystem of the sonotrode coincide with the flexural oscillation frequency of the annular element 33 with aperture 34 in the opposite part with respect to the tang.

Although a substantially circular, open annular element 33 is illustrated in the figures, it is obvious that the term annular element is intended to mean also an elliptical element or an element with similar geometry besides a circular element.

Numerous changes and modifications of detail, within the reach of a person skilled in the art, can be made to the present embodiments of the invention without thereby departing from the scope of the invention as set forth in the appended claims.

## Claims

1. An ultrasound resonant dipole (3) for medical applications comprising:
- a tang (30, 31, 32) designed to be connected to a handset comprising an ultrasonic transducer (1) that vibrates at ultrasound frequency with a longitudinal axis of vibration (X-X) coaxial with the axis of the tang,
- an annular element (33) connected to said tang, and
- a tool (5) disposed on said annular element (33) with an axis of vibration (Y-Y) inclined with respect to the axis of vibration (X-X) of the transducer,
**characterised in that**
- said annular element (33) is open or discontinuous and has an aperture (34) disposed along the continuation of the axis (X-X) of the transducer, in a position diametrically opposite the attachment of the tang to the annular element of the sonotrode.

2. A dipole (3) according to claim 1, **characterised in that** the axis of vibration (Y-Y) of said tool (5) is inclined, with respect to the axis of vibration (X-X) of said transducer, by an angle (ϑ) between 80° and 140°, preferably between 95° and 115°, mostly about 105°.

3. A dipole (3) according to any one of the preceding claims, **characterised in that** said annular element (33) has an outside diameter (C) of less than 20 mm, preferably between 10 mm and 16 mm.

4. A dipole (3) according to any one of the preceding claims, **characterised in that** said annular element (33) has a solid cross section with a thickness (D) between 2 mm and 8 mm, preferably between 3 mm and 6 mm.

5. A dipole (3) according to any one of the preceding claims, **characterised in that** said aperture (34) in the annular element has an outwardly increasing width.

6. A dipole (3) according to claim 5, **characterised in that** on the inside diameter of the annular element (33), the aperture (34) has a width (F) between 0.3 mm and 2.5 mm, preferably between 0.6 mm and 1.8 mm, whereas on the outside diameter of the annular element, the aperture (34) has a width (E) between 0.5 mm and 3 mm, preferably between 0.8 mm and 2 mm.

7. A dipole (3) according to any one of the preceding claims, **characterised in that** a flat surface (35) on which is disposed said tool (5) is provided on the outer surface of said annular element.

8. A dipole (3) according to claim 7, **characterised in that** said axis (Y-Y) of vibration of the tool (5) is at right angles with respect to said flat surface (35).

9. A dipole (3) according to any one of the preceding claims, **characterised in that** said tool (5) is a hammer which has a substantially cylindrical percussion part (50).

10. A dipole (3) according to claim 9, **characterised in that** said cylindrical percussion part (50) of the hammer has a diameter between 1.0 mm and 5.0 mm, preferably 3 mm.

11. A dipole (3) according to claim 9 or 10, **characterised in that** said hammer (5) has an overall length between 1.5 mm and 7.0 mm, preferably 3 mm.

12. A dipole (3) according to any one of claims 9 to 11, **characterised in that** said hammer (5) comprises a cylindrical or parallelepiped attachment part (51, 51') with a diameter or side smaller than the diameter of the percussion part (50).

13. A dipole (3) according to any one of the preceding claims, **characterised in that** said tool (5) is made in a single piece with said annular element (33).

14. A dipole (3) according to any one of claims 1 to 12, **characterised in that** said tool (5) is applied removably to an attachment of said annular element (33).

15. A dipole (3) according to any one of the preceding claims, **characterised in that** said tang of the sonotrode comprises a first part (30) with a square section, a second part (31) with a rectangular section with a smaller surface than the surface of the section of the first part and a tapered transition part (32) which is connected to the annular element (33).

16. A dipole (3) according to any one of the preceding claims **characterised in that** said tool (5) vibrates along its axis (Y-Y) at the same vibration frequency as the transducer (1) of the handset along its longitudinal axis (X-X).

17. A dipole (3) according to any one of the preceding claims **characterised in that** said transducer (1) of the handset vibrates along its longitudinal axis (X-X) at a frequency between 24 kHz and 30 kHz, preferably 27 kHz.

## Patentansprüche

1. Ultraschall-Resonanzdipol (3) für medizinische Anwendungen, umfassend:
- einen Zapfen (30, 31, 32), der zur Verbindung mit einem Handapparat konstruiert ist, umfassend einen Ultraschallwandler (1), der mit Ultraschallfrequenz längs einer Schwingungsachse (X-X) koaxial mit der Achse des Zapfens schwingt,
- ein ringförmiges Element (33), das mit dem Zapfen verbunden ist; und
- ein Werkzeug (5), das an dem ringförmigen Element (33) angeordnet ist, mit einer Schwingungsachse (Y-Y), die in Bezug auf die Schwingungsachse (X-X) des Wandlers geneigt ist,
**dadurch gekennzeichnet, dass**
- das ringförmige Element (33) offen oder unterbrochen ist und eine Öffnung (34) aufweist, die entlang der Fortführung der Achse (X-X) des Wandlers in einer Position angeordnet ist, die diametral gegenüber der Befestigung des Zapfens an dem ringförmigen Element der Sonotrode liegt.

2. Dipol (3) gemäß Anspruch 1, **dadurch gekennzeichnet, dass** die Schwingungsachse (Y-Y) des Werkzeugs (5) in Bezug auf die Schwingungsachse (X-X) des Wandlers mit einem Winkel (ϑ) zwischen 80° und 140°, vorzugsweise zwischen 95° und 115°, am meisten bevorzugt ungefähr 105° geneigt ist.

3. Dipol (3) gemäß einem der vorhergehenden Ansprüche, **dadurch gekennzeichnet, dass** das ringförmige Element (33) einen Außendurchmesser (C) von weniger als 20 mm, vorzugsweise zwischen 10 mm und 16 mm aufweist.

4. Dipol (3) gemäß einem der vorhergehenden Ansprüche, **dadurch gekennzeichnet, dass** das ringförmige Element (33) einen massiven Querschnitt mit einer Dicke (D) zwischen 2 mm und 8 mm, vorzugsweise zwischen 3 mm und 6 mm aufweist.

5. Dipol (3) gemäß einem der vorhergehenden Ansprüche, **dadurch gekennzeichnet, dass** die Öffnung (34) in dem ringförmigen Element eine nach außen zunehmende Breite aufweist.

6. Dipol (3) gemäß Anspruch 5, **dadurch gekennzeichnet, dass** am Innendurchmesser des ringförmigen Elements (33) die Öffnung (34) eine Breite (F) zwischen 0,3 mm und 2,5 mm, vorzugsweise zwischen 0,6 mm und 1,8 mm aufweist, wohingegen an dem Außendurchmesser des ringförmigen Elements die Öffnung (34) eine Breite (E) zwischen 0,5 mm und 3 mm, vorzugsweise zwischen 0,8 mm und 2 mm aufweist.

7. Dipol (3) gemäß einem der vorhergehenden Ansprüche, **dadurch gekennzeichnet, dass** eine flache Fläche (35), auf der das Werkzeug (5) angeordnet ist, auf der Außenfläche des ringförmigen Elements vorgesehen ist.

8. Dipol (3) gemäß Anspruch 7, **dadurch gekennzeichnet, dass** die Schwingungsachse (Y-Y) des Werkzeugs (5) in Bezug auf die flache Fläche (35) im rechten Winkel liegt.

9. Dipol (3) gemäß einem der vorhergehenden Ansprüche, **dadurch gekennzeichnet, dass** das Werkzeug (5) ein Hammer ist, der ein im Wesentlichen zylinderförmiges Schlagteil (50) aufweist.

10. Dipol (3) gemäß Anspruch 9, **dadurch gekennzeichnet, dass** das zylinderförmige Schlagteil (50) des Hammers einen Durchmesser zwischen 1,0 mm und 5,0 mm, vorzugsweise von 3 mm aufweist.

11. Dipol (3) gemäß Anspruch 9 oder 10, **dadurch gekennzeichnet, dass** der Hammer (5) eine Gesamtlänge zwischen 1,5 mm und 7,0 mm, vorzugsweise von 3 mm aufweist.

12. Dipol (3) gemäß einem der Ansprüche 9 bis 11, **dadurch gekennzeichnet, dass** der Hammer (5) ein zylinderförmiges oder quaderförmiges Befestigungsteil (51, 51') mit einem Durchmesser oder einer Seite kleiner als der Durchmesser des Schlagteils (50) aufweist.

13. Dipol (3) gemäß einem der vorhergehenden Ansprüche, **dadurch gekennzeichnet, dass** das Werkzeug (5) einstückig mit dem ringförmigen Element (33) hergestellt ist.

14. Dipol (3) gemäß einem der Ansprüche 1 bis 12, **dadurch gekennzeichnet, dass** das Werkzeug (5) lösbar an einer Befestigung des ringförmigen Elements (33) angebracht ist.

15. Dipol (3) gemäß einem der vorhergehenden Ansprüche, **dadurch gekennzeichnet, dass** der Zapfen der Sonotrode ein erstes Teilstück (30) mit einem quadratischen Querschnitt, ein zweites Teilstück (31) mit einem rechteckigen Querschnitt mit einer kleineren Fläche als der Fläche des Abschnitts des ersten Teilstücks und ein sich verjüngendes Übergangsteil (32) aufweist, das mit dem ringförmigen Element (33) verbunden ist.

16. Dipol (3) gemäß einem der vorhergehenden Ansprüche, **dadurch gekennzeichnet, dass** das Werkzeug (5) entlang seiner Achse (Y-Y) mit der gleichen Schwingungsfrequenz wie der Wandler (1) des Handapparats entlang seiner Längsachse (X-X) schwingt.

17. Dipol (3) gemäß einem der vorhergehenden Ansprüche, **dadurch gekennzeichnet, dass** der Wandler (1) des Handapparats entlang seiner Längsachse (X-X) mit einer Frequenz zwischen 24 kHz und 30 kHz, vorzugsweise 27 kHz schwingt.

## Revendications

1. Dipôle résonant à ultrasons (3) pour applications médicales comprenant :
- un tenon (30, 31, 32) conçu pour être raccordé à un appareil manuel comprenant un transducteur à ultrasons (1) qui vibre à la fréquence des ultrasons avec un axe de vibration longitudinal (X-X) coaxial avec l'axe du tenon,
- un élément annulaire (33) raccordé au dit tenon, et
- un outil (5) disposé sur ledit élément annulaire (33) avec un axe de vibration (Y-Y) incliné relativement à l'axe de vibration (X-X) du transducteur,
**caractérisé en ce que**
- ledit élément annulaire (33) est ouvert ou discontinu et a une ouverture (34) disposée dans la continuation de l'axe (X-X) du transducteur, dans une position diamétralement opposée à la fixation du tenon à l'élément annulaire du sonotrode.

2. Dipôle (3) selon la revendication 1, **caractérisé en ce que** l'axe de vibration (Y-Y) dudit outil (5) est incliné, relativement à l'axe de vibration (X-X) dudit transducteur, d'un angle (ϑ) compris entre 80° et 140°, de préférence entre 95° et 115°, de manière davantage préférée d'environ 105°.

3. Dipôle (3) selon l'une quelconque des revendications précédentes, **caractérisé en ce que** ledit élément annulaire (33) a un diamètre extérieur (C) inférieur à 20 mm, de préférence compris entre 10 mm et 16 mm.

4. Dipôle (3) selon l'une quelconque des revendications précédentes, **caractérisé en ce que** ledit élément annulaire (33) a une section transversale solide avec une épaisseur (D) comprise entre 2 mm et 8 mm, de préférence entre 3 mm et 6 mm.

5. Dipôle (3) selon l'une quelconque des revendications précédentes, **caractérisé en ce que** ladite ouverture (34) dans l'élément annulaire a une largeur augmentant vers l'extérieur.

6. Dipôle (3) selon la revendication 5, **caractérisé en ce que** sur le diamètre intérieur de l'élément annulaire (33), l'ouverture (34) a une largeur (F) comprise entre 0,3 mm et 2,5 mm, de préférence entre 0,6 mm et 1,8 mm, alors que sur le diamètre extérieur de l'élément annulaire, l'ouverture (34) a une largeur (E) comprise entre 0,5 mm et 3 mm, de préférence entre 0,8 mm et 2 mm.

7. Dipôle (3) selon l'une quelconque des revendications précédentes, **caractérisé en ce qu'**une surface plate (35) sur laquelle est disposé ledit outil (5) est fournie sur la surface extérieure dudit élément annulaire.

8. Dipôle (3) selon la revendication 7, **caractérisé en ce que** ledit axe (Y-Y) de vibration de l'outil (5) est à angle droit relativement à ladite surface plate (35).

9. Dipôle (3) selon l'une quelconque des revendications précédentes, **caractérisé en ce que** ledit outil (5) est un marteau qui a une partie de percussion sensiblement cylindrique (50).

10. Dipôle (3) selon la revendication 9, **caractérisé en ce que** ladite partie de percussion cylindrique (50) du marteau a un diamètre compris entre 1,0 mm et 5,0 mm, de préférence de 3 mm.

11. Dipôle (3) selon la revendication 9 ou 10, **caractérisé en ce que** ledit marteau (5) a une longueur globale comprise entre 1,5 mm et 7,0 mm, de préférence de 3 mm.

12. Dipôle (3) selon l'une quelconque des revendications 9 à 11, **caractérisé en ce que** ledit marteau (5) comprend une pièce de fixation cylindrique ou parallélépipédique (51, 51') avec un diamètre ou un côté plus petit que le diamètre de la partie de percussion (50).

13. Dipôle (3) selon l'une quelconque des revendications précédentes, **caractérisé en ce que** ledit outil (5) est formé en une seule pièce avec ledit élément annulaire (33).

14. Dipôle (3) selon l'une quelconque des revendications 1 à 12, **caractérisé en ce que** ledit outil (5) est appliqué de manière amovible à une fixation dudit élément annulaire (33).

15. Dipôle (3) selon l'une quelconque des revendications précédentes, **caractérisé en ce que** ledit tenon dudit sonotrode comprend une première partie (30) avec une section carrée, une seconde partie (31) avec une section rectangulaire avec une surface plus petite que la surface de la section de la première partie et une partie de transition conique (32) qui est raccordée à l'élément annulaire (33).

16. Dipôle (3) selon l'une quelconque des revendications précédentes, **caractérisé en ce que** ledit outil (5) vibre le long de son axe (Y-Y) à la même fréquence de vibration que le transducteur (1) de l'appareil manuel le long de son axe longitudinal (X-X).

17. Dipôle (3) selon l'une quelconque des revendications précédentes, **caractérisé en ce que** ledit transducteur (1) de l'appareil manuel vibre le long de son axe longitudinal (X-X) à une fréquence comprise entre 24 kHz et 30 kHz, de préférence de 27 kHz.
